Europäisches Patentamt

European Patent Office

Office européen des brevets

(19)

(11) Publication number: **0 048 470**
A2

# (12) EUROPEAN PATENT APPLICATION

(21) Application number: 81107426.9

(22) Date of filing: 18.09.81

(51) Int. Cl.³: **C 12 N 15/00**

(30) Priority: 22.09.80 US 189511

(43) Date of publication of application:
31.03.82 Bulletin 82/13

(84) Designated Contracting States:
AT BE DE FR GB IT LU NL

(71) Applicant: UNIVERSITY OF SOUTHERN CALIFORNIA
University Park
Los Angeles California 90007(US)

(72) Inventor: Baker, Robert Frank
607 Almar Avenue
Pacific Palisades Calif. 90272(US)

(74) Representative: Hansmann, Axel et al,
Albert-Rosshaupter-Strasse 65
D-8000 München 70(DE)

(54) Eucaryotically active recombinant DNA.

(57) Eucaryotic DNA replication origin sequences are used as cloning vectors in placing DNA sequences into eucaryotic cells. By a "shotgun" procedure, random DNA segments, connected to eucaryotic replication origin sequences, are transfected into transformed eucaryotic cells which are then selected for cells that have reverted to normal cell phenotype. From the reverted cells, plasmid DNA molecules are isolated and when attached to procaryotic plasmids are amplified in bacteria. The eucaryotic replication origin sequences are obtained by isolating scrambled and palindromic (i.e. inverted repeat) eucaryotic DNA segments, joining the palindromic segments to procaryotic plasmids, transfecting the set of recombinant DNA molecules into eucaryotic cells and selecting for surviving, replicating, supercoiled recombinant molecules. These molecules are replicated in bacteria and then isolated as supercoiled plasmids. Random DNA segments are preferably obtained from the genome of eucaryotic cells of the same phenotype as the phenotypically transformed cells and are scrambled by dividing and randomly joining the segments. Sets of these scrambled segments are attached to eucaryotic replicating origin sequences and used as reverter plasmids in transformed cells. Efficiency in changing cellular phenotypes is enhanced by incorporating additional palindromic eucaryotic DNA sequences, each at least 25 base pairs long. By appropriate survival selection techniques, reverter plasmids are obtained from tumorigenic cells and from virally infected cells. Reverter plasmids are potentially obtainable from other phenotypically altered eucaryotic cells, for example, cells containing DNA sequences which code for auto-immune disease, and cells manifesting the effects of aging.

./...

Croydon Printing Company Ltd.

FIG. 1

1

# EUCARYOTICALLY ACTIVE RECOMBINANT DNA

## FIELD OF THE INVENTION

The field of art to which the invention pertains includes the field of molecular biology, particularly genetic engineering.

## BACKGROUND AND SUMMARY OF THE INVENTION

Recombinant DNA molecules are constructed by splicing lengths of deoxyribonucleic acid (DNA) from different sources. One of the lengths can be a plasmid or virus cloning vector containing a procaryotic replication origin, i.e., a gene or genes that code for replication in a host bacterial cell. Bacterial plasmids are autonomously replicating extra-chromosomal elements found in many bacterial species. However, the term "plasmid" is also used in a broader sense to refer to any segment of DNA, linear or circular, which can replicate in a cellular environment. Particularly useful vectors are plasmids obtained from the bacterium Escherichia coli (E coli). common to the human intestinal tract. E coli species have been developed which are incapable of existing outside of a controlled, laboratory environment (Curtiss Patent 4,190,495) and

2

therefore provide a safe vehicle for manipulation and replication of recombinant molecules. The other length of DNA often termed a "passenger" segment, is selected to accomplish a desired genetic function.

Recent years have seen the development of a wide variety of techniques for the manipulation and expression of genetic information. Enzymes, called restriction endonucleases, have been discovered which cleave the DNA, each at specific recognition sequences. Mechanical and sonic shearing techniques have also been developed. Various techniques have been devised for joining DNA fragments. For example, enzymatic catalysts known as ligases are used to seal the joint between DNA pieces that are held together by cohesive terminii ("sticky ends") or by transient contact with the blunt ends of two DNA fragments. Techniques are available for converting cohesive ends into blunt ends and vice-versa. Effective methods have been devised for transfection, i.e., insertion of the recombinant plasmid, into either procaryotic cells or eucaryotic cells. Specialized plasmids have been developed containing markers for drug resistance which aid in selection and amplification. The positions of restriction sites on such plasmids have been mapped and the procaryotic replication origin located. Such plasmids can be accurately cut with an appropriate restriction endonuclease to insert a DNA fragment without destroying the replication origin or the site of desired drug resistance.

It is now becoming practical to splice known segments of DNA, either isolated as such or synthesized, to a procaryotic plasmid and insert the resultant plasmid into a host procaryotic cell where a large

3

number of cloned copies of the spliced DNA segment are produced. Through a process known as transcription, ribonucleic acid (RNA) may be produced corresponding to the spliced DNA segment which, in conjunction with ribosomes of the cell, may be translated into a specific protein, for example, insulin. In a related procedure, the DNA segments are obtained by "shotgunning" in which an entire eucaryotic genome is fragmentized and the pieces are attached to plasmids which are then randomly inserted into host cells. Colonies of host cells are grown and searched for a desired property. Such a procedure has recently been used for the synthesis of interferon.

A different category of use of recombinant DNA is to reinstruct existing somatic cells. This type of genetic engineering seeks to alter certain body cells to correct inborn errors, such as errors in metabolism, birth defects (Aposhian, "The Use of DNA for Gene Therapy -- The Need, Experimental Approach, and Implications," 14 Perspectives Biology Med. 98, 1970), and to solve problems such as cancer ("Recombinant Molecules: Impact on Science and Society" ed. by Beers, Jr. and Bassett, Raven Press, New York, 1976) and aging (Aposhian, supra, at 99).

A key component of a recombinant plasmid is its replication origin. As reported by Beach et al in "Isolation of Chromosomal Origins of Replication in Yeast", Nature, vol. 284, p. 185 (March 13, 1980), replication origins have been identified in the DNA of viruses, mitochondria, bacterial plasmids and bacterial chromosomes. Although Beach et al describe the constuction of a plasmid whose efficiency of replication in yeast is greatly enhanced by the insertion of certain fragments of yeast chromosomal DNA, believed to contain replication origins of the yeast, they state that the origins of replication of eucaryotic chromosomes have remained elusive because of the large size and sequence complexity of eucaryotic chromosomes and for the want of a suitable assay for their detection. It is also known to use the "early" gene of a simian virus (SV40) as a eucaryotic vector to join with a different eucaryotic or procaryotic DNA segment. (G.C. Fareed et al: "Cloning of a Segment from the Immunity Region of Bacterophage λ DNA in Monkey Cells", Recombinant Molecules, ed. R.F. Beers, Jr. and F.G. Bassett, Raven Press, New York (1977) pp. 299-316.) However, experiments have indicated that the function of the SV40 origin sequence requires the presence of the protein product of the SV40 A gene. This gene product is the T antigen which binds to the replication origin to obligatorily form part of the replication complex. Undesirably, the T antigen also causes many non-permissive cell types to become tumorigenically transformed.

Procaryotic plasmids, or their replication origins, do not generally replicate in eucaryotic cells. Therefore, in order to use selected DNA segments or procaryotic plasmids as parts of recombinant molecules in the somatic cells of an animal, one would have to provide each DNA segment or plasmid with a covalently attached eucaryotic replication origin sequence. Preferably, the eucaryotic replication origin will be "relaxed", i.e., over-replicating to provide a large number of copies per cell division. This can have the effect of stoichiometrically forcing recombination between new incoming DNA sequences bearing such relaxed origins and other or host chromosome DNA sequences. Generally, origins which serve to allow two or more copies per cell can be considered "relaxed" for the present purposes. The provision of such replication origins attached to appropriate DNA sequences would enhance the possibility for recombinant events that can alter the cell phenotype.

It would also be desirable to limit recombination so that it is effective to the extent necessary to revert a transformed cell to the normal or desired phenotype but not so as to excessively change the phenotype. For example, it is possible that some oncos genes for cancer are otherwise normal genes in which segments of the DNA sequence are transposed or are not contiguous in the non-cancerous cell. It would be desirable to limit the extent of recombinant activity to a retransposition of those segments, or changes, having the effect of countering only those transpositions. Similar considerations appear to be present with regard to other desirable phenotypic alterations.

The present invention provides the means for accomplishing the foregoing objectives. A method is provided for preparing a new form of cloning plasmid for eucaryotic cells useful in replication _in vivo_ of an _in vitro_ constructed plasmid DNA. The new plasmid allows the placing of new DNA sequences in eucaryotes with recombination and/or expression of new gene activity resulting from the presence of a new plasmid-borne DNA sequence. Methods are also provided to enhance the recombination efficiency of the plasmids with limited changes in phenotype. The method is specifically

6

exemplified by eucaryotically active recombinant DNA which when placed inside of cancer cells replicates and causes phenotypic changes such that the cancer cells are no longer transformed and are not tumorigenic. The method is also illustrated with recombinant DNA which acts as an anti-viral agent. The same procedure, in a more generalized form, would be useful against autoimmune diseases and diseases that are chromosome, episome and/or plasmid-caused or controlled. The method in its general form, with appropriate selection and survival procedures, has the potential to abate aging and revert its effects.

More particuarly, a method is provided for preparing eucaryotically active recombinant DNA. Eucaryotic replication origin sequences are joined to heterogenous DNA segments and, in one method, to procaryotic plasmids containing a drug-resistance marker, to form circular molecules of DNA serving as recombinant plasmids. By a "shotgun" procedure, these random recombinant plasmids are inserted into transformed eucaryotic cells and replicated. The eucaryotic cells are treated to suppress the transformed phenotype so as to select, as survivors, cells that have reverted to normal cell phenotype. Thereafter, the replicating reverters (self-selected recombinant plasmids) can be isolated from the survivor cells as supercoiled plasmids. If a drug-resistance marked procaryotic plasmid is used, the supercoiled plasmids can be inserted into procaryotic cells which are replicated and selected in accordance with the plasmid's drug resistance; the reverter plasmids are then amplified in the selected procaryotic cells and again isolated as supercoiled plasmids to constitute eucaryotically active recombinant DNA.

The preparation of eucaryotic replication origin sequences constitutes a significant advance in the art and is based on the isolation of specific sequences from eucaryotic cells. These sequences are in the form of substantially palindromic DNA segments. The term "palindrome" is a word used by the art and is specifically defined herein as referring to inverted repeat DNA sequences in which one branch is repeated in reverse with complementary bases of the other. Accordingly, they are not literal palindromes. Such sequences constitute about 3-5% of the total DNA in the genome (depending upon the particular type of eucaryotic DNA) and can be obtained by denaturing isolated DNA and allowing it to renature so that intrastrand hydrogen bonding of adjacent complementary inverted sequences of the same DNA strand will cause pairs of the sequences to fold back on themselves to form structures that have been referred to as hairpins ("The Origin and Complexity of Inverted Repeat DNA Sequences in Drosophila", R.F. Baker et al, DNA Insertion Elements, Plasmids, and Episomes, eds. Bukhari, Shapiro and Adhya, Cold Spring Harbor Laboratory, Cold Spring Harbor, New York, pp. 463-469) or as fold-back DNA ("A Study of Fold-Back DNA" by S. Pearlman et al, Cell, vol. 8, 33-42, May 1976). In one convenient method for selection of replication origin sequences, the palindromic eucaryotic DNA segments are joined to procaryotic plasmids, preferably together with a set of heterogenous DNA segments (this is found to enhance selection of the replication origins), followed by a selection procedure to isolate the plasmids containing eucaryotic replication origins. In this selection procedure procaryotic plasmids are used that have genes encoding resistance to a predetermined drug. The plasmids containing the palindromic eucaryotic segments are inserted into eucaryotic cells that are then replicated. Supercoiled plasmids are isolated from the replicated eucaryotic cells and those that have shown substantial replication are inserted into procaryotic cells that are replicated and selected in accordance with their drug resistance. Finally, the desired plasmids containing eucaryotic replication origin sequences are isolated as supercoiled plasmids from the drug-selected procaryotic cells.

Heterogenous DNA segments are used both in obtaining the eucaryotic replication origin and in the subsequent "shotgun" selection procedure. In accordance with another aspect of the invention, I have discovered that by obtaining these heterogenous eucaryotic DNA segments in accordance with a particular procedure, one can increase the possibility of useful recombination without excessive changes in phenotype. In this regard, I provide "scrambled" eucaryotic DNA segments obtained by successive steps of division, joinder and division. In particular, eucaryotic DNA is divided into first segments at first DNA breakage locations. The first segments are then randomly joined and then the joined sections are divided into other segments at DNA sequence locations different from the first locations. The multiple rounds of breakage and random reunion results in a scrambling of the DNA segments to provide internal sequences that are transposed relative to the original DNA sequence. In a particularly effective scrambling procedure, the DNA is cut with an endonuclease that is sequence non-specific and the cutting action is halted for successive increments over a period of time. The result is a broad spectrum of size ranges.

It is preferred, although not critical, to obtain the DNA from cells of the same phenotype as the phenotypically transformed eucaryotic cells. In this way, the chances for reversion of chromosome segments transposed in the transformed cells (compared with normal cells) is enhanced. Moreover, because the new segments are composed simply of transposed sequences, even though they are effective in reverting a particular phenotype abnormality, it is very unlikely that they will cause other phenotype changes.

Recombination between a scrambled DNA sequence inserted into a eucaryotic cell and the cell's chromosomal DNA is an event enhanced by the number of copies of a particular inserted DNA sequence. Prior selection and amplification of a sequence will allow a single type of sequence to be inserted into a eucaryotic cell. Accordingly a single phenotypic change can result without the possibility of other changes occurring. Moreover, the inserted sequence may be a relatively short sequence assembled from even shorter sequences by the scrambling process. As above indicated, selection of the proper scrambled sequence may be done by transfection of a random set of scrambled sequences into a population of eucaryotic cells. Selection for the desired phenotypic change among the progeny cells will automatically yield daughter cells carrying the desired scrambled sequence. This sequence will be present in multiple copies per cell as supercoiled plasmid DNA, provided that the original transfected DNA carries replication origin sequences on each of the different scrambled pieces in the set of ligated molecules.

In still another embodiment, I have found that recombinant efficiency is enhanced by joining a further DNA segment to the plasmid. This segment is also substantially palindromic, but no effort is made to isolate replication origins therefrom. In particular, a substantially palindromic eucaryotic DNA sequence, at least 25 base pairs long, is joined to the plasmid and can be added separately or at the same time as the scrambled DNA sequences are joined to the plasmids.

The cells whose phenotype is to be changed can be chosen so as to obtain eucaryotic activity with respect to a particular genetic malfunction. For example, the cells can be tumorigenic cells or cells susceptible to viral infection. Potentially, they can contain DNA sequences which code for autoimmune disease or they can manifest the effects of aging. A method is provided for causing a phenotypic change in eucaryotic cells comprising simply the step of inserting into the eucaryotic cells recombinant "reverter" DNA prepared in accordance with the above method.

The reverter DNA molecules may be treated _in vitro_ by appropriate encapsulation systems to protect the DNA from nucleases; for example, protein coats or membranes can be formed around the recombinant molecules. The encapsulated DNA molecules may then be put into whole animals or transfected into tissue culture cells. Alternatively, the recombinant molecules can be applied _in vitro_ to cells which are then transfused into the living animal, an older method of the art, as recently utilized in a particular way by Cline et al "Gene

11

Transfer in Intact Animals", <u>Nature</u>, vol. 284, pp. 422-425, April 3, 1980.

It will be appreciated that the above described methods yield novel compositions of matter. Thus, I provide a novel eucaryotic DNA molecule containing a substantially palindromic eucaryotic DNA segment serving as a eucaryotic replication origin sequence. Also novel are molecules containing an inserted recombinant DNA segment obtained by the aforedescribed steps of dividing, joining and dividing. Finally, a novel composition of matter has been provided in DNA having enhanced recombinant properties, comprising a recombinant plasmid and containing a substantially palindromic DNA segment at least 25 base pairs long.

### BRIEF DESCRIPTION OF THE DRAWINGS

FIGURE I is a flow chart diagrammatically outlining the principal method steps in preparing eucaryotically active recombinant DNA;

FIGURE 2 is a flow chart diagrammatically outlining the principal method steps in preparing eucaryotic replication origin sequences; and

FIGURE 3 is a flow chart diagrammatically outlining the principal method steps in alternative procedures for scrambling DNA segments.

### DETAILED DESCRIPTION

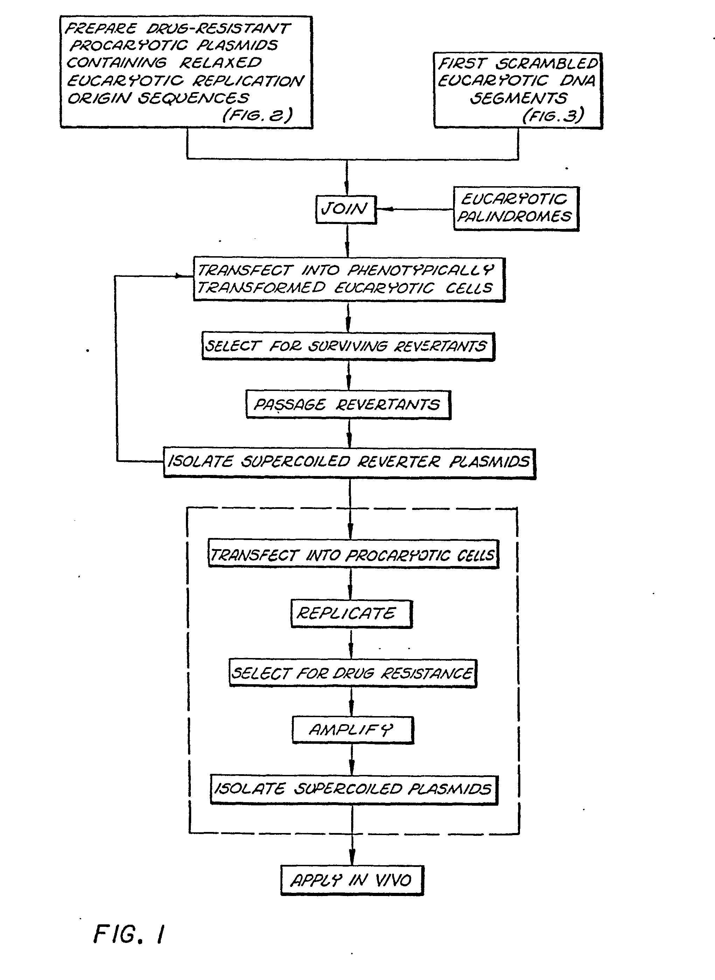

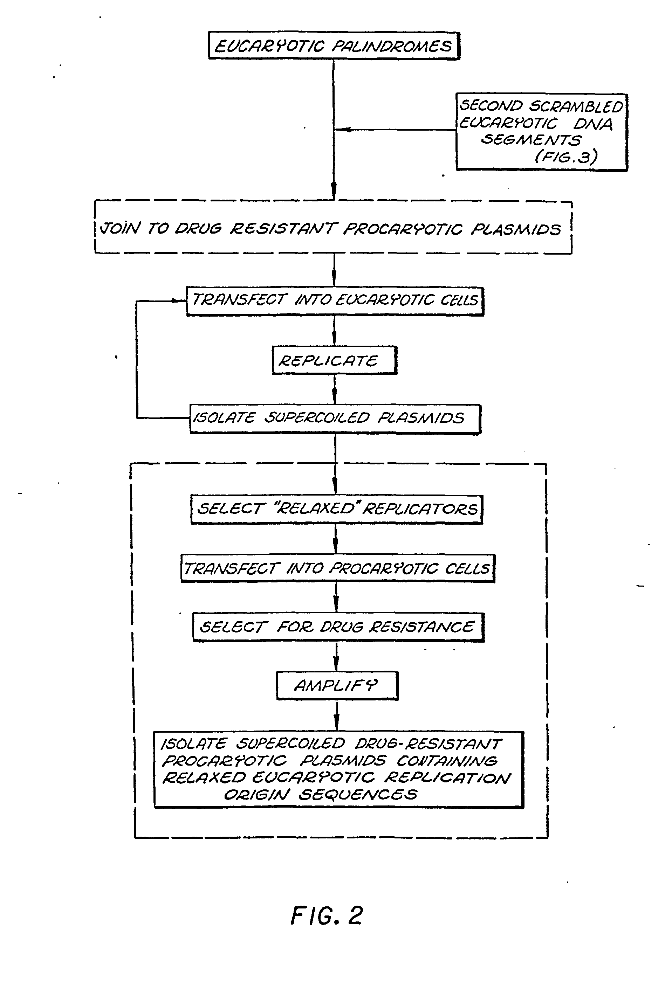

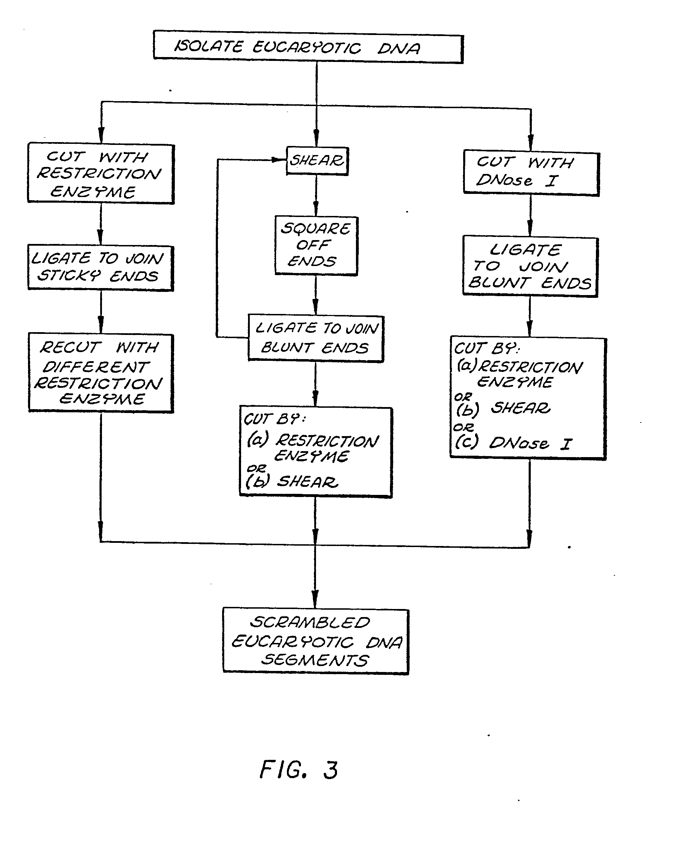

The figures present steps which taken together represent a specific method for preparing eucaryotically active recombinant DNA molecules. The initial steps of Fig. 1 call for preparing relaxed replication origin sequences containing drug-resistant procaryotic plasmids and then joinng these plasmids to "scrambled" eucaryotic DNA segments. Specific details for preparation of these components are given in Figs. 2 and 3. In Figs. 1 and 2 dashed lines or side boxes are used to indicate an optional step or sequence of steps. In particular, the dashed lines indicate the use of a drug-marked procaryotic plasmid, as a convenient means for selecting and amplifying.

Preparation of plasmids containing eucaryotic replication origin sequences

Referring to Fig. 2, the eucaryotic replication origin sequences are derived from eucaryotic palindromes, as hereinabove described, i.e., inverted repeat DNA sequences in which one branch is repeated in reverse with complimentary bases of the other. One starts with eucaryotic palindrome sequences obtained by any method known to the art; see for example D.G. Dickinson and R.F. Baker, Proc. Natl. Acad. Sci. U.S.A. (1978), vol. 75, 5627 where isolated eucaryotic DNA is sheared, diluted, denatured in alkali and allowed to renature in sodium phosphate solution, forming double-strand "hairpins". After isolation of the hairpins, for example, by means of hydroxyapetite, and several repetitions of the procedure, the palindromic sequences are ready to be either transfected directly into eucaryotic cells or, prior to that step, joined to suitable procaryotic plasmids. In either case, as indicated by the side box, scrambled, heterogenous, eucaryotic DNA segments are joined to the palindromic sequences by simple ligation, which may occur simultaneously with joinder to the procaryotic plasmids. The presence of the scrambled segments is found to enhance the selection of replication origins. They are prepared as shown in Fig. 3 and will be described in more detail hereinafter.

Joinder at this stage to a drug-resistant procaryotic plasmid provides a convenient means for selection and amplification. A large number of procaryotic plasmid vectors have been derived from E. coli and are known and available to the art. One of the smallest and most versatile plasmid vectors is PBR322 described in detail in H.W. Boyer et al: "The Construction of Molecular Cloning Vehicles", Recombinant Molecules, eds. R.F. Beers, Jr. and E.G. Bassett, Raven Press, New York, 1977, pp. 9-20. This plasmid contains two selective markers, one for ampicillin resistance ($A_p^r$) and the other for tetracycline resistance ($T_c^r$). Depending upon the particular endonuclease used to open the circular plasmic DNA, one or the other, or both, drug-resistance markers are retained on the plasmid following ligation to insert a foreign DNA segment. The positions of 36 restriction endonuclease sites cleaved by 11 different nucleases are mapped by the reference.

Any one of several well known methods to open the plasmid and join the foreign segments can be used. See, for example, R.J. Roberts: "The Role of Restricton Endonucleases in Genetic Engineering", Recombinant Molecules, eds. A.F. Beers, Jr. and E.G. Bassett, Raven Press, New York (1977) pp. 21-32. Boyer et al, supra, at p. 10, describes three methods for ligating DNA molecules in vitro. See also F. Bolivar and K. Backman: "Plasmids of Escherichia coli as Cloning Vectors", Methods in Enzymology, vol. 68, 1979, pp.

247-252 and N.P. Higgins and N.R. Cozzarelli: "DNA-Joining Enzymes: A Review", Methods in Enzymology, vol. 68, 1979, pp. 50-71. Specifically, I have derived eucaryotic replication origin sequences from TC7 African green monkey kidney cell DNA and cloned them onto PBR322 using EcoRI and BamHI to open the PBR322 circles within the Ap gene. The recombinant DNA can be precipitated in alcohol solution, and centrifuged to a pellet form. The pellets can be dried and then solubilized for transfection into cells or other manipulations.

Transfection of the recombinant plasmid into eucaryotic cells can be accomplished using procedures known to the prior art. (In this regard, the prior art has sometimes used the word "transformation" to describe such insertions, but since this specification describes phenotypic changes as transformations, the word "transfection" will be used to indicate cell insertions.) More particularly, a calcium phosphate transfection method is used, in which the pelleted DNA is dissolved in a sterile, slightly alkaline solution. Calcium chloride is then added along with a buffered solution of $Na_2HPO_4$ and NaCl. The DNA forms a suspension with calcium phosphate crystals which is applied to a plate or flask containing eucaryotic cells. In an alternative transfection procedure a solution of diethylaminoethyl dextran and phosphate-buffered saline solution can be added to the DNA to facilitate transfection of the eucaryotic cells.

The transfected eucaryotic cells replicate and are "passaged", a procedure whereby the attached cells are treated with trypsin to loosen the cells. The cells are diluted with phosphate-buffered saline solution,

15

propagated to cover the bottom of a flask or plate, again treated with trypsin and diluted as part of repeated cycles of dilution, propagation, and trypsinization.

The plasmids of interest are supercoiled and can be isolated by procedures based on the method of Hirt (B. Hirt: "Selective Extraction of Polyoma DNA from Infected Mouse Cell Cultures", J. Mol. Biol. (1967), vol. 26, 365-369), in which cellular DNA from lysed cells is preferentially precipitated in the presence of sodium dodecyl sulphate and NaCl. The precipitate is centrifuged and supercoiled plasmids are precipitated from the supernatent with cold ethanol. At this stage, one will have evidence of whether or not the plasmid contains a relaxed eucaryotic replication origin. In those cases where no replication origin has been joined to the plasmid, or where the replication origin is "stringent" (i.e., yielding fewer than two copies of plasmid per cell division), precipitation of supercoiled DNA from the supernatant Hirt solution will be very light. A heavy precipitation indicates the presence of plasmids having relaxed eucaryotic replication origin sequences. As indicated in Fig. 2, it is the relaxed plasmids that are selected.

The selected plasmids can be retransfected into eucaryotic cells and the process of passaging and isolation repeated several times, each cycle increasing the relative amount of eucaryotically relaxed plasmid until stringent- or non-replicating plasmids are an insignificant portion of the isolated DNA. It will be appreciated that the above procedure, by definition, selects for plasmids containing relaxed eucaryotic replication origin sequences. As a result, the final recombinant DNA molecule will abundantly accumulate when inserted into the target animal cell; replication in vivo in tissue culture cells is at a rate and efficiency such that the plasmids are present in excess of 40 copies per nucleus on a steady state basis.

Still referring to Fig. 2, the relaxed replicators are next transfected into procaryotic cells, such as E. coli bacterial cells. The procedure of Cohen et al can be followed (Cohen et al: "Nonchromosomal Antibiotic Resistance in Bacteria: Genetic Transformation of Escherichia coli by R-Factor DNA", Proc. Nat. Acad. Sci. U.S.A, vol. 69, pp. 2110-2114.) in which the plasmids are added to a suspension of the bacterial cells in a dilute calcium chloride solution which upon low temperature incubation and then higher temperature incubation, results in transfection of the procaryotic cells which are then plated onto appropriate nutrient agar or grown in liquid culture.

The plated procaryotic cells are then selected for drug resistance by culturing with the appropriate drug, in this case ampicillin, and are then amplified in fresh media containing the antibiotic. In this regard, the cells accumulate a great number of plasmid DNA copies per cell under conditions where protein synthesis and chromosomal DNA synthesis have ceased, as described by Hershfield et al (V. Hershfield et al: "Plasmid Col E1 as a Molecular Vehicle for Cloning and Amplification of DNA", Proc. Nat. Acad. Sci. U.S.A. (Sept. 1974), vol. 71, No. 9, pp. 3455-3459.) When amplifying, the media is shaken to grow the bacteria and chloramphenicol is added to a final concentration of 250 micrograms per ml of media. The cells can then be compacted by centrifugation for storage as pellets at subzero temperature. The drug-resistant procaryotic plasmid is isolated from the E. coli cells as supercoiled plasmids. Following the procedure of Clewell and Helinski: "Supercoiled circular DNA-protein complex in Escherichia coli: Purification and induced conversion to an open circular form", Proc. Nat. Acad. Sci., U.S.A. (1969), Vol .62, p.p. 1159-1166, the cells are lysed, added into an equal volume of Brij solution (containing 1% Brij 58, 0.4% sodium deoxycholate, 0.0625 molar ethylenediaminetetraacetate, and 0.05 molar tris buffer, pH 8) and the solution is centrifuged. The plasmids are recovered by phenol extraction of the supernatant and concentrated by ethanol precipitation followed by further phenol extraction, the phenol being removed by dialysis or by ether extraction to yield the drug-resistant supercoiled procaryotic plasmids containing relaxed eucaryotic replication origin sequences.

Scrambled eucaryotic DNA segments.

As indicated in Figs. 1 and 2, palindromic segments are joined to scrambled eucaryotic DNA segments to form eucaryotically active procaryotic plasmids and the plasmids themselves are joined to scrambled segments. Fig. 3 indicates alternative methods for obtaining such scrambled segments, essentially by processes involving successive division, joinder and division.

The use of scrambled segments constitutes another significant advance in the art. Multiple recombination events allowing the translocation of DNA segments within a genome allow an almost endless variation in the creation of nucleotide sequence diversity. Starting with a long genomic DNA molecule, the degree of diversity of new sequences that can be created is determined by (a) the randomness of double strand breaks, (b) the number of breaks per genomic length, and (c) the randomness of reunion (translocation) of the fragments. The use of the word "genome" here has two implications: (1) Evolution has created at least the minimum number and kinds of sequences that allow a set of functions to occur. The set of functions may include those that are the result of translocations of sequences in vivo. (Any one DNA molecule may represent the order of internal sequences at the time the molecule was isolated.) (2) The nucleotides in the DNA sequences are arranged to have an order that specifies information. Rearrangement of sections of the sequence can lead to the specification of new information. The translocation of one short sequence to a new position in a genome could, for example, create a new phenotype for cells carrying this genome. On the other hand, breakage of a - finite-length DNA molecule into mononucleotides followed by the nucleotides being relinked in a random order would seemingly result in DNA molecules having very few and then only short length-sequences carrying genetic information.

Thus, in order to usefully scramble a DNA genome, it is necessary to break the DNA into pieces with a wide distribution of lengths and to do so with a randomness to the double strand break positions. Joining of random ends of these pieces will allow scrambling of the genomes. If a large number of similar genomes (a large amount of DNA) simultaneously participate in this breakage and reunion system, a high diversity of new combinations of sequences will result; the newly created junctions between pieces will be novel.

Accordingly, a significant feature of the scrambling technique is its ability to randomly join pieces having a wide distribution of lengths. In this way not only are a wide array of new junctions created, but also the lengths of sequence adjacent to new junctions are allowed to vary over the considerable range that may be designed into the experiment. If three pieces are joined, the piece in the middle may theoretically be any piece of any given length in the original genome.

If more than one piece of DNA (scrambled pieces or unscrambled pieces) is transfected into a cell, there is the opportunity for recombination to occur in vivo between a pair of the incoming sequences. Such recombination events between participating DNA pieces allows further scrambling to occur between sequences of similar or even different genomes.

It is possible to use DNAs from different cell types (including species) or different viruses or combinations thereof to create, either in vitro or in vivo, even further new sequences by these scrambling methods. From the almost infinite variety of new sequences that may be transfected into large populations of cells, selection of the desired biologically active scrambled DNA sequence is accomplished by selection methods described hereinafter where the desired DNA sequence survives and is replicated in particular cells within the population.

All three processes shown in Fig. 3 use ligase

20

to attach pieces of DNA together _in vitro_ in a solution. On the left in Fig. 3, the eucaryotic DNA to be scrambled is cut or broken into pieces by one or more site specific procaryotic restriction enzymes.

A large number of endonucleases are known to the art, each active with respect to a particular DNA sequence, typically from 4 to 6 base pairs long. Many of these endonucleases (such as EcoRI, BamHI, Hae III, etc.) will cleave the double-stranded DNA molecules at offset positions so as to produce "sticky ends"; see R.J. Roberts, _supra_.

The reaction conditions for these enzymes vary with the type of enzyme. For instance, EcoRI and BamHI are used at 37°C in a solution containing 50 mM NaCl, 20 mM Tris, pH 7.7, 10 mM $MgCl_2$, 10 mM 2-mercaptoethanol; Hae III conditions are the same except that the NaCl is excluded. Following this step, ligation is performed at 12-22°C using T4 ligase in the presence of 50 mM NaCl, 20 mM Tris, pH 7.7, 10 mM $MgCl_2$, 10 mM 2-mercaptoethanol, 80 micromole ATP. Multiple rounds of restriction followed by ligation allows additional scrambling if a different restriction enzyme cutting at a different nucleotide sequence is used each time. It will be appreciated that the pieces generated by a particular restriction enzyme have identical sticky ends, therefore the ligation results in the formation of DNA strands having randomly transposed segments. These strands are then recut with a different site-specific endonuclease restriction enzyme to produce what I have termed "scrambled" eucaryotic DNA segments.

Referring now to the center of Fig. 3, a physical method of "scrambling" is indicated. In this method, DNA is sheared by mechanical means such as sonication or by being driven through a hypodermic syringe needle. Sonication, using commercially available laboratory sonicators, will break pieces of DNA into lengths no shorter than about 500 base pairs on the average; hypodermic syringe needles, in which the DNA solution is driven through the needle by maximum thumb pressure on the attached syringe, gives pieces longer than about 2000 base pairs. Next, the ends of the segments are squared off, which can be readily accomplished using S1 nuclease digestion (Case and Baker, "Investigation into the Use of Aspergillus Oryzae S1 Nuclease in the Presence of Solvents which Destabilize or Prevent DNA Secondary Structure: Formaldehyde, Formamide, and Glyoxal", _Analytical Biochemistry_, Vol. 64, 1975, p.p.477-484). The squared-off segments are then joined by blunt end ligation techniques as herein above described. To increase transposition, one can repeat the process several times, in each case shearing, squaring off the ends and ligating, as indicated by the recycle arrow. Thereafter, depending upon the technique that one wishes to use for joining the scrambled segments to the eucaryotically active plasmid, one can either shear one more time followed by S1 nuclease treatment or one can cut the strands with a restriction endonuclease.

The right hand side of Fig. 3 shows a most useful method for scrambling DNA segments produced by cutting the long isolated eucaryotic DNA with an endonuclease sequence that is non-specific. This method has the advantage that the pre-ligation pieces are randomly cut from the eucaryotic genome and that short pieces, even on the order of 20 to 50 base pairs, may be obtained and then scrambled. As described in more detail hereinafter, the method provides a very high degree of permutation of the DNA providing a wide spectrum of short-to-long segments. The segments have blunt ends and are ligated then reset by restriction enzymes, by shearing, or again by DNase I to yield the scrambled DNA segments.

More particularly, pancreatic DNase I is used in the presence of 10 mM Tris buffer, pH 7.7, and 0.6 to 0.7 mM $MnCl_2$, 20° to 30°C. Under these conditions DNase I largely causes double strand breaks, rather than single strand nicks (Melgar, E. and D. A. Goldthwait (1968) Deoxyribonucleic Acid Nucleases II. The Effects of Metals On the Mechanism of Action of Deoxyribonuclease, T. J. Biol. Chem. 243, 4409-4416). Depending upon the amount of enzyme added to a given amount of DNA, the reaction volume, the time period of the reaction, and the temperature, different length pieces of DNA can be obtained as short as even 10 base pairs. Ligation of the pieces into a random order is done by the action of T4 DNA polymerase and also S1 nuclease to increase the yield of pieces that are blunt ended and T4 ligase to connect the blunt-ended pieces.

This method provides a system for making a continuous distribution of lengths of DNA pieces which have been cut at random sites. DNA under the previously described enzyme conditions is mixed with DNase I at time zero. Starting at this time, the DNA solution is gradually pumped over a 30 minute period from the reaction vessel into another vessel containing ethylene diamine tetraacetate (EDTA) solution. The EDTA solution stops the enzyme reaction by chelating the manganese ions. The result of this operation is that a diminishing proportion of the DNA in the reaction vessel is acted upon the the 30 minute maximum time of the reaction. The earliest DNA pumped into the EDTA has the longest pieces; the last DNA pumped contains the shortest pieces. Thus a continuous distribution of sizes of DNA is produced and is ready for ligation.

The proportion of longer pieces to shorter pieces in the continuous distribution of lengths produced by pumping may be shifted by adding greater or lesser amounts of enzymes. Another technique is to gradually mix the DNA into the enzyme solution while at the same time gradually pumping the mixture into an EDTA enzyme-stopping solution. This technique may be accomplished using a two chambered gradient maker in which stirring is done in the chamber containing the enzyme solution. If the stirring chamber contains the DNA and the enzyme is gradually allowed to mix with the DNA (the reverse of the above mixing method), then the product DNA that gradually accumulates in the EDTA solution will have a different but still continuous distribution of longer pieces to shorter pieces. The value of these continuous-distribution methods is that virtually every recombination of sequence can then be achieved during the ligation.

It is possible to use more than one kind of DNA in these scrambling systems. For instance an evolutionarily lower eucaryotic DNA mixed initially with a higher eucaryotic DNA will allow the scrambling of sequences that are highly evolved with some that may have been lost in evolution. One utility of this operation is the retrieval of combinations of sequences that could have biologically active consequences when transfected into present cells.

24

## Additional palindromes.

Referring back to Fig. 1, the scrambled eucaryotic DNA segments are joined to the relaxed eucaryotic replication origin sequences, for example as carried by drug-resistant procaryotic plasmids. Such joinder can be by any ligation method appropriate to the method of preparation of the scrambled segment and generally following the procedures hereinabove given. As shown by the side box, at the same time, or subsequently, or even prior to such joinder, one can add additional eucaryotic palindromic segments. These palindromic segments are not selected on the basis of yielding eucaryotic replication origin sequences but are randomly selected hairpins. They are isolated as was the eucaryotic palindromes of Fig. 2 and have ends formed as appropriate to the ligation mixture to which it is going to be joined. Of course, if joined prior or subsequent to ligation of the scrambled segments, any convenient procedure can be utilized. The palindromic sequences that are used at this stage are at least 50 bases (i.e., 25 base pairs) long and typically are in the range of 100-2000 bases (i.e., 50-1000 base pairs). Although the reasons are not fully understood, I have found that the presence of these inverted repeat sequences on the plasmid greatly increases the probability of breakage and reunion recombination events between the recombinant plasmid and the host chromosome. Accordingly it is desirable to use both scrambled DNA molecules and palindromic molecules in constructing reverter molecules.

Survival-selection to obtain reverters.

The result of the foregoing procedure is a "shotgunned" mixture of recombinant plasmids, the great majority of which will not be useful for the reversion process to be conducted, but a small amount may contain a recombinant segment that is transposed just enough to effect a reversion of the undesired phenotype to a desired phenotype. The next steps in the process are designed to select those plasmids, to serum diluted to 1%; the result is a negative selection of the normal cells.

More specifically, the transfected eucaryotic cells initially are cultured in a medium containing 10% fetal calf serum so that all the cells, both tumorigenic and normal cells, grow in the serum. Thereafter, the cells are cultured in 1% fetal calf serum which is too dilute to culture normal cells but which is sufficient for the growth of tumorigenic cells. 5-Bromodeoxyuridine is then added, the mixture is incubated and the cells are removed from the medium and exposed to ultraviolet light which along with the 5-bromodeoxyuridine serves to kill the tumorigenic cells. The remaining cells are then incubated in 10% fetal calf serum, containing very small amounts of scavengers for the 5-bromodeoxyuridine (such as thymidine and uridine). The cells are allowed to grow for several weeks, with a change in the medium daily so as to remove the dead cells. One can then isolate the plasmid DNA from the surviving cells as supercoiled plasmid using the Hirt extraction method described above with respect to Fig. 2.

An alternative selection procedure, somewhat more direct than the above procedure is based upon the use of the lectin concanavalin A which distinguishes between normal and transformed cells by agglutinating, at concentrations of concanavalin A which do not affect normal cells, and preferentially killing the transformed cells (B. Ozanne: "Variants of Simian Virus 40-Transformed 3T3 Cells that are Resistant to Concanavalin A", Journal of Virology, vol. 12, No. 1, July 1973, pp. 79-89). In this procedure, the transfected phenotypically transformed cells are cultured to a density of about 3 X 10$^4$ cells/cm (as determined by hemocytometry). The cells are then treated with concanavalin A to kill the tumorigenic cells.

In either case, the surviving revertants are passaged using the same passaging procedure described above with respect to Fig. 2. After washing with phosphate-buffered saline solution and incubation for several weeks, supercoiled recombinant DNA plasmids are isolated from the supernatant obtained by the Hirt procedure as described above. The plasmids are amplified by recycling several times through transfection, selection and isolation steps to obtain high frequency reverters.

It will be appreciated that surviving cells will be "normal" for one of three reasons: (1) they are normal to begin with; (2) they reverted naturally from the tumorigenic state; or (3) they reverted by an event involving the recombinant DNA. Plasmid from only the last type of cell is of interest and is amplified by being subjected to recycling several times through transfection, negative selection and isolation. By definition, the recombinant plasmid obtained from the survivor cells will be active in causing reversion. It will also be appreciated that if reversion succeeds as a result of an exchange or recombination of the passenger DNA segment with a DNA sequence in the transfected host cell, because the plasmid is of the relaxed type, the bulk of the isolated recombinant plasmids will comprise copies of the DNA that are capable of, but have not yet, exchanged or recombined their passenger segment.

The high frequency reverter molecules may then be cloned by transfection into procaryotic cells such as E. coli. In this regard, E. coli transfection, selection, amplification and isolation procedures described above with respect to Fig. 2 are fully applicable here. Accordingly, the plasmids are added to a suspension of the procaryotic cells in a dilute calcium chloride solution which is incubated at low temperature and then incubated at higher temperature. Thereafter, the transfected procaryotic cells are plated onto nutrient agar or grown in liquid culture. The procaryotic cells are then selected for drug resistance by culturing with the appropriate drug and amplified in fresh media containing the antibiotic. The media is shaken to grow the bacteria and chloramphenicol is added. The cells can then be concentrated by centrifugation for storage as pellets at subzero temperature. The drug-resistant procaryotic plasmid is isolated from the procaryotic cells as supercoiled plasmids by lysing with a Brij solution, and recovery by phenol extraction of the supernatant followed by further phenol extraction, the phenol being removed by dialysis or by ether extraction to yield the drug-resistant supercoiled procaryotic plasmids, which may be concentrated by ethanol precipitation.

28

Isolated supercoiled plasmid recombinant DNA obtained following the above procedures has been found to cause a high  frequency of reversion of a second set of transformed cells.  In particular, using DNA obtained from African green monkey kidney cells, when several rounds of transfection, reversion and isolation have been practiced, the resultant plasmid DNA has yielded recombinant plasmid that has reverted monkey, mouse and human cancer cells at a high frequency.  In the case of human cells, retransformation of the reverted cells by human papovavirus BK does not occur.

Generally while it is not desired nor intended to be limited by any particular theory of operation, it can be hypothesized that the phenotype is converted to the normal state by alteration or rearrangement of a small portion of the DNA genome, regardless if transformation as the neoplastic state of a cell line resulted from either covalent or noncovalent changes in the genome or even metabolic changes in the cell.  Conversion of the phenotype could be a direct effect or an indirect effect, i.e., a suppression effect on pleiotropic phenotypes.  When a stem cell, i.e., an original cell, becomes transformed, a covalent change in the DNA (e.g., a DNA sequence translocation or DNA methylation pattern change), a non-covalent change (e.g., a regulator protein or hormonal effect) or no change in the DNA could have occurred.  By definition, certain DNA sequences which are important to the transformed state are present on the virally or carcinogenically transformed cell's genome; all progeny cells that contain the transformed phenotype must carry replicas of these DNA sequences.  Microbiologists currently debate whether there are many states of transformation that a cell line may go through on its time course excursion to a final transformed state. The observation is that many phenotypic changes occur, some sequentially, during the process of transforming a cell line. The argument against there being individual genetic loci for each transformation phenotype is that a cell may be defensively countering the act of being transformed. Although the debate has not been resolved, the outcome of the above procedure has not depended upon the number of genetic loci involved in transformation being one or even a very few. Apparently, if the number of loci is greater than one, then at least small sections of the loci as DNA sequences are similar, or the alteration of a few DNA loci by recombination with an incoming, replicating DNA plasmid results in a cascade effect on the regulation of, or gene products involved in, transformation. This latter effect could even involve pleiotropic suppression of multiple phenotypic traits that are expressed in the transformed state.

Other uses.

The foregoing procedures can also be applied to obtain plasmids that will serve as antiviral drugs to prevent virally caused lytic and cytopathic effects. When a eucaryotic virus infects a cell, the virus may lie dormant as a so-called latent virus, it may transform the cell as in the case of a tumor virus infecting a non-permissive cell, or it may lyse a permissive cell. There are certain constraints on both RNA and DNA viruses as to their host range in each of the three general examples of effects given above. However, even short of killing the host cell by lysis, there is a wide spectrum of so-called cytopathic effects. I have found that it is possible to prevent (before a virus infection) or counter (within a short time after a virus infection) the effects of certain viral infections. This has been done by the use of recombinant DNA molecules prepared in much the same manner as previously described for the making of transformed cell reverter molecules. The difference in obtaining the antiviral DNA plasmids is largely in the selection process.

More particularly, antiviral DNA has been obtained by transfection of tissue culture, such as TC7 African green monkey kidney cells, with the collection of scrambled sequence and palindrome-bearing DNA plasmids that also contain replication origin sequences. This may be done before or after infection by a virus such as SV40 for TC7 cells, which will cause cytopathic effects and lysis of the cells. Those few particular cells which acquire protection from the effects of the virus have been changed in phenotype by a small portion of the transfected DNA molecules. Selection for antiviral DNA molecules is based on the fact that they are harbored in the surviving cells. Of this population of cells (which are allowed to grow) a small portion will have the desired self-replicating antiviral plasmid DNA molecules.

The methodology for obtaining these selected molecules is as previously described for the reverter molecules. In this regard the previously described steps are followed except that it is preferable (although not critical) to isolate the DNA to be scrambled from eucaryotic tissue culture cells that are infected with the virus of interest. The extent of viral infection can be determined using plaque assay techniques and microscopic observation as known to the art. After the genomes from the infected cells have been scrambled and the DNA segments ligated to an excess of drug-resistant plasmid containing eucaryotic replication origin sequences, the resultant DNA molecules are transfected into virally uninfected eucaryotic tissue culture cells which are then infected with the target virus. Those cells that survive the viral infection are passaged and replicating reverter plasmid DNA is obtained from the supernatant of a Hirt extraction procedure, following the procedures described above. The reverter plasmids are cycled twice through the transfection, selection, passage and extraction steps to isolate the reverters. These DNA molecules may then be cloned into E. coli via the drug resistance marker on the procaryotic plasmid portion of the molecule. This step and subsequent steps of selection in accordance with drug resistance, amplification and isolation as supercoiled plasmids are as described with respect to Fig. 1.

While the procedures of the present invention have been exemplifed with tumorigenic and virally infected cells it will be appreciated that the present techniques have broad application. DNA passenger fragments can be obtained from a variety of sources and can be subjected to a variety of survival-selection techniques. The procedures thus have the potential for preparing eucaryotically active DNA to: (a) change cell surface proteins; (b) stop autommmune diseases (such as multiple sclerosis and lupus erythematosus) by DNA recombination in vivo; (c) select and insert interferon genes into eucaryotic cells at high gene dosage levels; (d) modulate growth factors; (e) negate undesirable genomic functions by recombination of plasmid and host sequences in vivo; and (f) reverse the effects of aging by selecting for autonomously replicating plasmids which revert aging in tissue culture cells by forced recombination in vivo. Techniques for in vivo recombination are indicated by M. Wigler: DNA Mediated Tansfer of the Adenine Phosphoribosyl-transferase Locus into Mammalian Cells", Proc. Natl. Acad. Sci., Vol. 76, No. 3 (March 1979), pp. 1373-1376, and by F.L. Graham and A.J. Van Der Eb: "A New Technique for the Assay of Infectivity of Human Adenovirus 5 DNA", Virology, Vol. 52 (1973), pp. 456-467.

Certain underlying considerations will serve to indicate the breadth of potential use for the procedures described herein. Cells contain DNA nucleotide sequences which are not directly biologically active because specific sequences must be contiguous for a derived longer sequence to be operational. Translocation events to achieve operational sequences may be performed in vivo by the cell (as during a cell differentiation event) or in vitro by the techniques described here. The described in vitro techniques should allow a far greater diversity of biologically active sequences than the natural in vivo translocations. The in vitro methods do not impose constraints upon the order, length, number, or permutation of the combined sequences. The in vivo translocations have constraints imposed by nature as a result of evolution and natural cell differentiation, with the exception of accidents and interventions from outside of the cell.

Scrambled sections of genomes bear contiguous sequences which when actively replicated in vivo, allow recombinations between existing natural chromosomal sequences and the incoming, transfected, scrambled sequences. A relatively short region of homology on the incoming molecule and a section of chromosome DNA will allow recombination to occur. The non-homologous (compared to a section of the scrambled incoming DNA) sequences to the left or right of the recombinationally active homologous sequence on the incoming DNA can be involved in illegitimate recombination events such that as an outcome, for example, a section of the chromosome to the left or right of the region of homology is altered. This genotypic or DNA change, which may be selected as phenotype change (as in the examples previously given for the antiviral and transformed cell reverter DNA molecules), may be permanent in the chromosomal DNA in subsequent daughter cells. The effect may be present in all of the similar chromosomal DNA sequences in the cells (e.g., in diploid and polyploid cells).

Examples of the utility of this method can be seen in the potential for altering of surface antigens on cells and the elimination of self recognition of proteins and other antigens in animals. The latter case has been suggested to be at the heart of the cause of autoimmune diseases such as multiple sclerosis.

The DNA vectors derived as carrying mammalian DNA replication origin sequences can also be used to place new genes into existing somatic cells. The gene of interest is ligated onto the DNA vector and the combination is transfected into tissue culture cells. The gene may have been previously obtained free from other sequences or selected _in vivo_ after transfection from the "shotgunned" pieces of a genome known to carry

34

the desired gene. In either case, the transfection would also allow the derivation of plasmid which may then be encapsulated as previously decribed for introduction of the new gene into the whole animal. Selection of interferon or similar antiviral genes may be accomplished by the same methods previously described for obtaining an operationally different class of self-replicating antiviral DNA molecules. Such self-replicating antiviral DNA molecules thus have utility as therapeutic drugs.

It will be apparent that the techniques described herein and the eucaryotically active recombinant DNA molecules will have wide ranging utility. An immediate utility is in medicine, both for humans and veterinarian, to overcome genetically or virally caused disorders. Reverter molecules are intrinsically useful in changing the phenotype of transformed-tumor producing cells. The work described here has utilized tissue culture cells for both selection and application of the reverter molecules. It is expected that these molecules will be useful in reverting the phenotype of neoplastic cells in humans and other animals. Reverter DNA molecules could be vectored into whole animals by encapsulation of the DNA prior to application to the animal. This could be done by surrounding the DNA by membrane material (e.g., the DNA placed inside of liposomes) or protein coats. In the latter case the DNA may be placed inside of a virus coat which is acquired by self-assembly of isolated virus coat proteins around the reverter DNA in vitro or as pseudovirions in vitro in which a virus infection of cells harboring reverter molecules causes some of the progeny virus to encapsulate the reverted DNA rather than the virus DNA.

Alternatively, the reverter plasmid DNA may be modified to include genes for the making of virus coat proteins. After the initial transfection of the naked reverter DNA molecules into cells, transcription and translation of the virus coat protein genes would allow the production of virus-like particles which can be obtained by standard methods known to the art of virology.

It should be appreciated that the material surrounding a reverter DNA molecule may act to protect the DNA from nucleases (particularly in the case of the use of the DNA in whole animals) and to target the DNA to particular tissues. In addition the coat will given the eucaryotically active recombinant DNA certain host ranges in being active not only in certain tissues, but in certain species of animals. By appropriate choice of coat, one is allowed a broadening or a narrowing of host range and vector-associated variables, such as tissue specificity.

Another utility is the use of cultured populations of eucaryotic cells in vitro, or even in live animals, such as mice, in particular athalmic mice, as hosts for the synthesis of interferon, hormones such as insulin, or growth hormone, etc. Because of the greater similarity of a host eucaryotic cell to the species through which the hormone is being synthesized, a product would be obtainable having closer similarities to the natural host hormone and therefore greater natural effectiveness.

Of course, the procedures of recombinant materials described herein have great utility in furthering recombinant DNA research.

36

The following specific examples will further illustrate the procedures.

## Example I

Recombinant reverters for tumorigenic cells were prepared as follows:

### Tissue Culture Cells

BKHFB12 and BKHFB12A human fetal brain cells, TC7 African green monkey kidney cells, and 3T3 and SV3T3 mouse embryo cells were grown in 75 cm$^2$ tissue culture flasks in Dubecco's modified Eagle's medium containing 5 or 10% fetal calf serum. Passages from one flask to the next at near confluency was done by detaching the cells from the flask surface by first washing the attached cells with 10-20 ml of phosphate buffered saline (PBS) [containing 0.14 M NaCl, 0.003 M KCl, 0.008 M Na$_2$HPO$_4$ 7H$_2$0, 0.0015 M KH$_2$PO4] followed by the addition of 2 ml of trypsin-EDTA solution [containing 0.05% (w/v) trypsin, 0.56 mM EDTA in PBS] and incubation of the cells at 37°C for a few minutes, until the cells visibly detach. The trypsinized cell suspension was forced several times through a Pasteur pipet to free the cells from clumps and then diluted with fresh medium into a T flask. The dilution per passage was normally one part cell suspension to four - eight parts medium; the dilution ratio depended upon the desired density of cells and the time desired between passages. Propagation of the different cell lines was continuous in that as confluency within a plate or flask was reached, dilution of the cell concentration after trypsinization allowed continued cell reproduction. These cell dilutions also served to dilute any originally transfected DNA (see below) that entered the cells but did not replicate or adventitiously bound DNA that placed onto the cells during transfection, but did not enter the cells.

Isolation of bulk DNA from tissue culture cells

At near confluency, the medium covering attached cells in 150 mm diameter x 15 mm plastic petri dishes was aspirated and the cells were washed twice with PBS solution. Ten ml of 0.1 M Tris, 0.1 M EDTA, pH 7.5 - 8 was added to each plate of cells, followed by sodium lauryl-sulfate to 0.5% (w/v) final concentration. After 10 minutes incubation at room temperature, the cell lysate was transferred to a 250 ml Erlenmeyer flask and pronase was added to a concentration of 1 mg per ml of lysate. The mixture was incubated overnight at 37°C and then additional pronase (0.1 mg per ml of lysate) was added to the lysate and incubation continued at 37°C for 6 to 16 hours. Ribonuclease A was added to a concentration of 10 micrograms/ml (from a stock solution containing 1 mg RNase per ml in 0.15 M NaCl, heated to 85°C for 10 minutes) and the 37°C incubation continued for an additional hour. The lysate was extracted three times with freshly distilled phenol that is equilibrated with 0.1 M Tris, 0.1 M EDTA, pH 7.5 - 8. The resulting DNA solution was then dialized against 10 mM Tris, 1 mM EDTA, pH 7.5 - 8.

Isolation of palindromic DNA

Tissue culture cells were cultured in Dulbecco's Modified Eagle's Medium containing 5% fetal calf serum and their DNA was isolated as described above. The DNA was sheared or enzymatically cut (see scrambling method below) to an average size of 50-3000 base pairs, as determined by electrophoresis on polyacrylamide (T. Maniatis  et al Biochemistry Vol. 14 (1975) pp. 3787-3794) or agarose gels. The sheared DNA was denatured in 0.3M NaOH and renatured in 0.15M Na phosphate, pH 6.8 at 25°C. After batch mixing with hydroxyapatite, the DNA bound at 0.15M and eluted at 0.4M Na phosphate, was collected, dialyzed against 0.1M NaCl, precipitated at -20°C with 2 volumes of ethanol. The DNA, containing inverted repeat sequences, was solubilized in 10 mM Tris, 1 mM EDTA, pH 7.7.

Conversion of double stranded DNA to hairpin DNA at palindrome sites

Bulk DNA (100 micrograms in 1 to 10 ml of 10 mM Tris, 1 mM EDTA, pH 7.5 - 8) was heated to 100°C in a boiling water bath for 20 minutes. The DNA was quick-cooled in an ice bath and used directly in the replication origin - cell phenotype change experiments. When the concentration of the DNA is greater than about 0.5 micrograms/ml (here it is used at 10 to 100 micrograms/ml) there is a considerable amount of bimolecular renaturation and lattice formation. However, the hairpin formation is concentration independent (Baker and Thomas, supra.) and the reaction product contains the hairpin DNA as well as single stranded and other renatured double strand DNA.

Scrambling sections of longer DNA sequences

100 micrograms of eucaryotic DNA in 10 mM Tris, 1 mM EDTA, pH 7.8 was precipitated by the addition of 0.1 volume of 3 M sodium acetate, pH 6.5 and 2 volumes of -20°C ethanol, held for 5-20 minutes in a dry ice - ethanol bath and pelleted by centrifugation at 15,000 xg for 5 minutes. The pellet was dried by placing the centrifuge tube in a vacuum dessicator and then dissolved in 10 ml of 10 mM Tris, pH 7.8, 0.70 mM

$MnCl_2$. Pancreatic DNase I ($10^{-10}$ to $10^{-5}$ grams per 10 ml of the above solution in 10-fold differences in enzyme concentration in separately pumped reactions as described below) was added and mixed with the DNA solution at time zero. The DNA/enzyme solution at 25°C was then slowly pumped, using a peristaltic pump with flexible tubing, into a second vessel containing 1 ml of 0.25 M´EDTA, pH 7.5. The second vessel contained a magnetically driven rotating mixing bar. The pumping was continuous, from the time of addition of the DNase I, at the rate of 1 ml per 3 minutes. After finishing the pumping, the DNA in the second vessel was precipitated by the addition of 0.1 volume 3 M solution acetate, pH 6.5 and 2 volumes of -20°C ethanol. The mixture was held in a dry ice - ethanol bath for 30 minutes and the DNA was pelleted in a plastic centrifuge tube by centrifugation for 30 minutes at 15,000 xg. The DNA pellet was dried by vacuum and then washed 3 times with 1 ml of 70% ethanol and then dissolved in 1.0 mM Tris, 0.1 mM EDTA, pH 7.5 - 8. The DNA solution was then heated to 68°C for 10 minutes. One tenth volume of 3 M sodium acetate, pH 6.5 and two volumes of -20°C ethanol were added. After 30 minutes in a dry ice - ethanol bath, the DNA was pelleted at 15,000 xg for 15 minutes, the pellet dried as before, and then dissolved in 1.0 ml of 0.03 M sodium acetate, pH 4.7; NaCl was added to 0.2 M, and $ZnCl_2$ added to $5 \times 10^{-4}$ M. $S_1$ nuclease (300 units) was added and the mixture incubated at 37° for 30 minutes (Case and Baker, "Investigation...", supra.) 0.25 M stock EDTA was added to 100 mM and the mixture was heated to 68°C for 10 minutes, 3 M stock sodium acetate was added to 0.3 M. and 2 volumes of -20°C

40

ethanol added with mixing. After 30 minutes in a dry ice - ethanol bath, the DNA was pelleted at 15,000 xg for 15 minutes. The pellet was dried and dissolved in 20 mM Tris, pH 7.8, 10 mM $MgCl_2$, 10 mM mercaptoethanol. Then NaCl was added to 50 mM, ATP to 80 micromole, and 4 units of T4 ligase were added and the mixture incubated at 16°C for 18 hours.

Construction of reverter molecules.

After the eucaryotic replication origin sequences are obtained as plasmids, these circles are cleaved by DNase I in the presence of 10 mM Tris, pH 7.7, 0.6 to 0.7 mM $MnCl_2$, 50 micrograms of bovine serum albumin per ml of reaction volume (but restriction endonuclease could alternatively be used, under standard conditions). The DNA segments are made blunt ended by S1 nuclease (described above, under Scrambling....) and the open circles are blunt-end ligated to the assortment of palindrome and scrambled DNAs, using T4 ligase in the presence of 50 mM NaCl 20 mM Tris, pH 7.7, 10 mM $MgCl_2$, 10mM 2-mercaptoethanol, 80 micromole ATP. The collection of ligated molecules (i.e., random palindromes and scrambled sequences ligated to an excess of replication origin sequences) are then transfected into the semiconfluent transformed cells growing in SV3T3 mouse tissue culture. (BKHFB12 and BKHF12A human fetal brain cells and transformed TC7 African green monkey cells have also been used.) Transfection is performed using the calcium phosphate crystal method to allow DNA to enter the cells (Graham, F. L. and A.J. Van der Eb (1973), "A new technique for the assay of infectivity of human adenovirus 5 DNA", supra; Parker, B.A. and G.R. Stark, "Regulation of Simian Virus 40 Transcription: Sensitive Analysis of

41

the RNA Species Present Early in Infections by Virus or Viral DNA", 2. Virology 31, pp. 360-369; Frost E. and L. Williams (1978) "Mapping temperature sensitive and host-range mutations of adenovirus type 5 by marker rescue", Virology 91, pp. 39-50).

DNA transfection of tissue culture cells

DNA was prepared by the scrambling process followed by isolation of the plasmid PBR322 from E. coli, isolation of bulk DNA from tissue culture cells, denaturation of bulk DNA followed by renaturation to form hairpin DNA (palindromes), etc. Following construction and ligation to form a set of DNAs to be introduced into the tissue culture cells (see above), 10 to 20 micrograms of DNA (including high molecular weight carrier DNA) was precipitated by the addition of 0.1 volume of 3 M sodium acetate, pH 6.5, followed by 2 volumes of $-20°C$ ethanol. After 30 minutes in a dry ice – ethanol bath, the DNA was pelleted by centrifugation at 15,000 xg for 15 minutes in a plastic tube. The supernatant was removed and the now alcohol-sterilized DNA pellet dried under sterile conditions in a vacuum dessicator. The pellet was then dissolved in 0.375 ml of 1 mM Tris, 0.1 mM EDTA, pH 7.8. 0.125 ml of 1 M $CaCl_2$ was then added and the mixture slowly added to 0.5 ml of 280 mM NaCl, 50 mM HEPE, 1.5 mM $Na_2HPO_4$, pH 7.1, by bubbling air through a cotton plugged 1 ml pipet. The mixture was allowed to stand for 30 – 45 minutes to allow calcium phosphate crystals to form. The precipitate was then gently mixed and added to semiconfluent media-free cells in a tissue culture plate or flasks. The cells were left at room temperature for 20 minutes and then 10 ml of DMEM with 5% FCS was added. After incubation at 37° in a $CO_2$ incubator for 4 hours, the media was removed and 2 ml of autoclaved 15% (v/v) glycerol, 0.15 M NaCl, 0.025 M HEPE, pH 7.4 was added. After 3 minutes at 37°C, the cells were washed once with approximately 5 ml of DMEM with 5% FCS. Fresh media was then added according to the selection procedure for the survival of the desired cell type harboring the desired plasmid type, as described below.

Obtaining supercoiled DNA from tissue culture cells

At the time that cells have been passaged at least four times after transfection, a Hirt supernatant (Hirt supra.) is prepared. This supernatant contains the supercoiled plasmid DNA and some chromosomal DNA: A 150 mm plate of semiconfluent cells is rinsed once with PBS (see above). Then 4 ml of 0.6% sodium lauryl sulfate, 0.02 M EDTA, 0.01 M Tris, pH 7.5 is added and the plate allowed to stand at room temperature for 20 minutes. The viscous lysate is transferred to a plastic centrifuge tube and 0.25 volume (usually 0.9 ml per 150 mm plate) of 5 M NaCl is added. The tube is covered with Parafilm and inverted 10 times to gently mix. After allowing the mixture to stand 4°C overnight, it is centrifuged 40 to 60 minutes at 25,000 xg at 4°C. The supernatant is taken, heat treated RNase (85°C) is added to 10 micrograms/ml final concentration and the mixture heated to 37°C for 30 minutes. Then one volume of phenol (previously equilibrated with 0.1 M Tris, 0.1 M EDTA, pH 7.8) is added and the mixture extracted once. The phenol is separated from the DNA solution by centrifugation at 5,000 xg for 10 minutes. The DNA solution layer, obtained after this centrifugation, is taken and is mixed with 0.1 volume of 20% potassium acetate, pH 6.5. and then 2 volumes of -20°C ethanol is added. After mixing and storage at -80°C overnight, the DNA was pelleted by centrifugation for 30 minutes at 10,000 xg. The pellet is dried and then dissolved in 1 mM Tris, 0.1 mM EDTA, pH 7.8.

Selection of cells reverted from the transformed state

Transformed cells (BKHFB12, BKHFB12A, SV3T3, etc.) were grown to approximately one-third to one-half confluency on 150 mm plastic tissue culture plates using Dulbecco's modified Eagle's medium (DMEM) containing 5 or 10% fetal calf serum at 37°C in an atmosphere containing 6% $CO_2$. After transfection of the cells with a population of DNA molecules as described above, the cells were selected for a reverted phenotype in order to enrich for the proper plasmids. Some of the populations of transfected DNA molecules have both the capacity to replicate as plasmids and to revert transformed cells.

Two methods have been used to enrich for cells carrying plasmids with these two properties. In the first method, the transfected cells in a plate were allowed to grow in DMEM with 10% fetal calf serum for 24 hours. The medium was then replaced with DMEM containing 0.9 to 1.0% FCS. After 24 hours 5-bromodeoxyuridine (BUdR) was added to a final concentration of 200 micrograms/ml of medium. After further growth for 96 hours, the lid of the plate was removed under sterile conditions and the medium covering the cells aspirated. The cells were immediately exposed to UV light (General Electric Germicidal Lamp) for 2 to 4 seconds, depending upon the cell line. Calibration of the killing effect of the UV light was previously done such that for a particular cell line with BUdR incorporated into the cells, 50% of the cells were killed. Fresh DMEM with 10% fetal calf serum was added to the UV-treated cells. This latter medium also contained $10^{-5}$ thymidine and usually $10^{-4}$ M uridine. The surviving cells were allowed to grow out in the 150 mm diameter plate. Hirt supernatants were prepared from these cells as described and the supercoiled DNA obtained was purified away from detergent and protein (also described elsewhere herein). This DNA was used to transfect transformed cells where again subsequent selection of revertants was done and supercoiled DNA obtained. Multiple rounds of transfection and cell selection allowed retrieval of supercoiled DNA which, compared with that obtained after the first transfection, was vastly more efficient at causing reversion of the transformed state. Three rounds of transfection with supercoiled DNA isolation allowed an increase in the rate of reversion from the natural background reversion rate (on the order of one cell in $10^5$ cells for most transformed tissue culture lines) to about 90% of the limiting step in these test procedures; this step is the limitation imposed by the efficiency of transfection itself.

Cells reverted from the transformed state were also selected as survivors in DMEM medium with 5 to 10% FCS and containing the lectin concanavalin A (con A) at 300 micrograms/ml of medium (Inbor, M. and Sachs, L., 1969. "Interaction of the carbohydrate-binding protein concanavalin A with normal and transformed cells," Proc. Natl. Acad. Sci. USA 63, 1418-1425). After 24 hours of selection in the con A-containing medium, the detached cells were washed away with sterile PBS and fresh medium was added to the surviving anchored cells. These cells formed colonies which were allowed to proliferate. Hirt supernatants were obtained from the cells as described and used to retransfect transformed cells. Each successive round of transfection followed by selection of revertants allowed enrichment for Hirt supernatant DNA containing supercoils acting as reverters; the concanavalin A method for selection allowed enrichment for reverters comparable to that previously described for the BUdR, UV light selection method.

The method of Hirt, (supra) is used to obtain the DNA enriched for supercoiled plasmids. This plasmid DNA is further purified by phenol extractions and dialysis against 10 mM Tris, 1 mM EDTA, pH 7.7. Buoyant density centrifugation of the DNA still further purifies the plasmid DNA away from the residual chromosomal DNA. This centrifugation is done at 37,000 rpm in a Beckman Spinco 50 Ti rotor at 18°C for 48 to 60 hours after adding 0.97 grams of CsCl to each ml of DNA solution which has been made 250 microgram/ml concentration in ethidium bromide. The plasmid DNA bands in the gradient of CsCl created during centrifugation. This band is of higher density than the chromosomal DNA; both bands can be visualized in the centrifuge tube by UV light. The lower (plasmid DNA) band is obtained and extracted three times with isopropanol saturated with CsCl in order to remove the ethidium bromide. After dialysis against Tris/EDTA buffer, the plasmid DNA is available for retransfection into tissue culture cells.
Cloning, amplification, and isolation of eucaryotically active recombinant DNA molecules.

Three rounds of retransfection, each time followed by isolation of the plasmid DNAs, are done to

46

increase the reversion ability of the reverter DNA as well as its biological stability. Homogeneity of certain acquired plasmids is achieved by attachment of the set of reverter-enriched DNAs to a procaryotic plasmid, in this case PBR322. Transfection of the procaryotic-eucaryotic recombinant DNA into E. coli allows selection of individual types of reverter DNA molecules. This technique is done by transfection of the DNA in the presence of calcium ion (Cohen et al, supra.) into E. coli followed by selection for antibiotic resistant bacteria; PBR322 carries genes for causing bacteria to be resistant to tetracycline and ampicillin. The eucaryotic pieces are routinely inserted into the tetracycline gene of PBR322 using conventional methods known to the art. This allows the selection of ampicillin-resistant bacteria having the recombinant DNA pieces.

Transfection of E. coli by DNA

Bacteria were transfected by the method of Cohen et al. (Proc. Natl. Acad. Sci. 69, 2110 (1972) with slight modification: E. coli HB101 cells, recA⁻, were grown to mid-log-phase by adding 0.1 ml of a fresh overnight culture to 4.3 ml of L broth in a 6 inch culture tube and shaking at 37°C for approximately 2 hrs or until the light absorbance in a Klett colorimeter with a red Klett filter was 30 - 40 units. The cells were chilled to 0 - 4°C in an ice-water bath and then pelleted by centrifugation at 6,000 xg, for 15 minutes. The pellet was washed once in 0.5 ml of cold 10 mM NaCl and the cells were repelleted. The cells were resuspended in 2.5 ml of cold 0.03 M CaCl₂, kept at 0°C for 20 minutes, and then again pelleted by centri-

47

fugation. The cells were resuspended in 0.44 ml of cold 0.03 M $CaCl_2$. 0.2 ml of this competent cell suspension was combined with 0.1 ml of DNA solution using a cold pipet. The DNA solution contained the appropriate DNA at 2 micrograms/ml and was 0.02 M Tris, pH 8.0, 1 mM EDTA, 0.02 M NaCl, 0.03 M $CaCl_2$. The transfected cell suspension was held at 0°C for 60 minutes and then heated to 42°C for 2 minutes prior to being diluted to 3 ml with 37°C L broth. The culture was allowed to incubate at 37°C for one hour and then either plated onto appropriate nutrient agar containing 20 micrograms/ml ampicillin for selection of ampicillin resistant colonies or added to 150 ml of L-broth containing 25 micrograms/ml ampicillin (in a 2 liter Erlenmeyer flask) for incubation at 37°C and selection and amplification of the transfected plasmid DNA. This amplification process, in which chloramphenicol is added to the L-broth culture, is described below. The presence of ampicillin in the plates and liquid cultures insures that only drug-resistant E. coli will grow and harbor the ampicillin resistance gene-bearing plasmid DNA molecules. L-broth contains Bactro-tryptone (Difco) 10% (w/v), yeast extract (Difco) 5% (x/v), NaCl 5% (w/v) glucose 1% (u/v); the pH is adjusted to 7 with 1 M NaOH prior to autoclaving. Plates contained L-broth with 1.5% agar (Difco).

Isolation of Supercoiled Plasmid DNA from E. coli

E. coli HB101 that had been transfected with DNA or E. coli HB101 carrying PBR322 plasmid DNA was grown in L broth in a 150 ml culture in a two liter Erlenmeyer flask at 37°C with shaking. When the bacterial growth reached $5 \times 10^8$ cells per ml of medium, chloramphenicol was added to a final concentration of 250

48

microgram/ml and growth with shaking continued for 5 hours. The cells were then pelleted by centrifugation at 6,000 xg for 20 minutes in a Sorvall RC2B refrigerated centrifuge. The cells were washed once with 0.15 M NaCl, 10 mm Tris, 1 mm EDTA, pH 7.5 - 8, repelleted, and frozen. The pellet was thawed and resuspended in 8 ml of 25% (w/v) sucrose in 50 mM Tris, pH 8.0. In an ice water-bath, 1.6 ml of 15 mg/ml lysozyme in 0.25 M Tris, pH 8.0 was added, followed by 3.2 ml of 0.25 M EDTA, pH 8.0. The mixture was allowed to stand at 4°C for 5 minutes and then pipeted into 12.8 ml of 1% Brij 58 detergent, 0.4% sodium deoxychloate, 0.0625 M EDTA, pH 8.0, 0.05 M Tris, pH 8.0. The solutions were mixed by covering with Parafilm and inverting ten times and then allowed to stand at 4°C for 10 minutes followed by an incubation for 3 minutes at 45°C. The lysate was then centrifuged at 11,000 xg for 1 hour in the RC2B centrifuge. The supernatant was removed and extracted three times with phenol that is neutralized prior to use with 0.1 M Tris, 0.1 M EDTA, pH 8.0. One tenth volume of 3 M sodium acetate, pH 6.5 was added and the DNA precipitated with two volumes of ethanol by holding the mixture for 30 minutes in a dry ice-ethanol bath. The DNA was pelleted by centrifugation at 15,000 xg for 20 minutes. The pellet was dried by vacuum and then dissolved in 7ml of 10 mm Tris, 1 mM EDTA, pH 7.5 - 8 prior to banding the DNA in CsCl-ethidium bromide as described below.

Isolation of supercoiled DNA on cesium chloride-ethidium bromide gradients.

The DNA solution from Hirt supernatants or Brij lysates was made 250 micrograms/ml in ethidium

49

bromide by the addition of sufficient stock (2 mg/ml) ethidium bromide solution. Solid CsCl was then added at 0.97 g CsCl per ml of the DNA/ethidium bromide solution and adjusted to give a density of 1.5644 g/cc with a refractive index of 1.38685. The solution was then centrifuged in a Beckman Spinco 50 Ti rotor at 18°C for 36 - 48 hours at 36 - 38 Krpm. The supercoiled DNA was visualized as a lower band in the CsCl gradient by the use of UV light. The gradient was franctionated and the ethidium bromide extracted from the supercoiled DNA by shaking the DNA solution with an equal volume of isoamyl alcohol that had been previously equilibrated with water and solid CsCl. After three separate extractions, the DNA was dialized against 10 mM Tris, 1 mM EDTA, pH 7.8.

Criteria for reverted cells

A reverted cell should be unable to grow in DMEM containing 1% fetal calf serum and be unable to form colonies in medium containing soft agar or soft methylcellulose (Macpherson, I. and L. Montagnier, 1964, "Agar suspension for the selective assay of cells transformed by polyoma virus", Virology 23, pp. 291-299; Freedman, V. and S. Shin 1974 "Cellular tumorigenicity in nude mice: Correlation with cell growth in semi-solid medium", Cell 3, p. 355). In animal studies the inability to form tumors in athymic ("nude") mice is a criterion of reversion of the transformed phenotype.

Testing reverted cells for transformed phenotype

Transformed cells grown in tissue culture usually form tumors when placed in the corresponding animal. This is particularly true when the animal is an athymic mouse. In the laboratory, the test with the

50

best correlation *in vitro* for tumor causing activity by cells *in vivo*, is the plating of cells in soft agar or soft methycellulose. The transformed cells will grow to form colonies while suspended in the soft matrix; the non-transformed or reverted cells do not grow. This method is also used for isolation of transformed cells from a mixed population. This method was used to test populations of transformed cells for the yield of reverted cells. In either 60 mm or 100 mm plastic petri dishes, the anchorage requirement was determined by diluting trypsized cells and then plating a dilution series in DMEM with 5 or 10% FCS containing 1.2% methylcellulose (Methocel, 4000 cps, Dow Chemical Co.) over a layer of 0.9% agar (Difco, Bacto Agar) also in DMEM with 5 or 10% FCS (Stoker et al., 1968, *Inter. Natl. Jour. Cancer*, **3**. 683-693; Shin et al., 1975, *Proc. Natl. Acad. Sci.*, **72**, 4435-4439). Alternatively, soft agar was used to test for anchorage dependence (Macpherson, I. and Montagnier, L., 1964, *Virology 23*, 291-294). In this method, 4 ml of Autopow minimal essential medium (Flow Laboratories) with 10% tryptose phosphate buffer containing 0.6% Difco Noble agar were poured into 60 mm plastic petri plates. This layer was overlaid with 1.5 ml of 0.5% agar containing the cells to be tested.

EXAMPLE II

Example I relates to the reversion of transformed cells; in this example, antiviral DNA molecules are constructed to be used as self-replicating drugs.

Selection for viral-resistance-conferring eucaryotic plasmid DNA

Two types of experiments have been performed which allow selection of plasmid DNA molecules conferring

51

viral resistance on cells prior to or immediately after a viral infection. One type of experiment utilizes tissue culture cells that are permissive for certain viruses. Normally the virus infection causes lysis of these cells. The selection procedure here is for cells that survive (are not lysed) and carry replicating plasmid supercoiled DNA molecules that confer this resistance. The other type of experiment utilizes tissue culture cells that are non-permissive for certain viruses, but are transformed as a result of the viral infection. The selection procedure in this case is for cells that are not transformed by the virus (if the transfection by the DNA is prior to viral infection) or are reverted from transformation (if the transfection by the DNA is after the viral infection).

Lytic systems used are SV40 virus infecting TC7 African green monkey kidney cells, and polyoma virus infecting 3T3 mouse cells, In these experiments the infections were done at 10 plaque forming units per cell. Otherwise, the procedures are as given above.

52

## THE CLAIMS

1. A method for preparing eucaryotically active recombinant DNA, comprising:

preparing DNA molecules containing eucaryotic replication origin sequences;

joining first heterogenous DNA segments to said eucaryotic replication origin segments to form recombinant molecules;

inserting said recombinant molecules into phenotypically transformed eucaryotic cells whereby to replicate therein;

treating said recombinant molecule-containing cells to suppress said transformed cells whereby to select, as survivors, cells that have reverted to normal cell phenotype; and

isolating and replicating reverter molecules from said survivor cells whereby to constitute said eucaryotically active recombinant DNA.

2. The method of claim 1 in which said eucaryotic replication origin segments are jointed to procaryotic plasmids, said procaryotic plasmids having genes encoding resistance to a predetermined drug, and in which the step of isolating and replicating reverter plasmids comprises;

isolating supercoiled reverter plasmids from said survivor cells;

inserting said reverter plasmids into procaryotic cells;

replicating said inserted reverter plasmids;

selecting said reverter plasmid-containing procaryotic cells in accordance with their resistance to said drug;

amplifying said reverter plasmids in said selected procaryotic cells; and

isolating supercoiled reverter plasmids from said selected procaryotic cells.

3. The method of claim 1 or 2 in which said eucaryotic replication origin sequences are chosen to impart relaxed replication properties to said recombinant molecules, and said DNA molecules containing eucaryotic replication origins are obtained by selection from first substantially palindromic eucaryotic DNA segments joined with second heterogenous DNA segments.

4. A method for preparing eucaryotically active DNA molecules serving as procaryotic cloning vectors, comprising joining first substantially palindromic eucaryotic DNA segments to procaryotic plasmids and selecting from the resultant plasmids those plasmids containing eucaryotic replication origins serving to impart relaxed eucaryotic replication properties to said plasmids.

5. The method of claim 1 or 4 in which said procaryotic plasmids have genes encoding resistance to a predetermined drug, and wherein the step of selecting plasmids containing eucaryotic replication origins comprises:

inserting said resultant plasmids into eucaryotic cells;

replicating said plasmid-containing eucaryotic cells;

isolating supercoiled plasmids from said replicated eucaryotic cells;

54

inserting said supercoiled plasmids into procaryotic cells;

replicating the resultant procaryotic cells;

selecting said resultant procaryotic cells;

selecting said resultant procaryotic cells in accordance with their resistance to said drug;

isolating from said selected procaryotic cells, supercoiled plasmids containing eucaryotic replication origin sequences; and

selecting those supercoiled plasmids, isolated from said replicated eucaryotic cells, that have shown substantial replication whereby to constitute the plasmids that are inserted into procaryotic cells.

6. A method for preparing eucaryotically active DNA molecules, comprising joining first substantially palindromic eucaryotic DNA segments to heterogenous eucaryotic DNA segments and selecting from the resultant molecules those molecules containing eucaryotic replication origins serving to impart relaxed eucaryotic replication properties to said plasmids.

7. A method for preparing eucaryotically active DNA molecules having enhanced recombinant properties, comprising joining to said molecules substantially palindromic DNA sequences at least 25 base pairs long.

8. The method of claim 7 in which said palindromic sequences are eucaryotic.

55

9. A method for preparing scrambled DNA segments, comprising:

dividing DNA into first segments at first DNA sequence locations;

joining said first segments; and

dividing said joined segments into other segments at DNA sequence locations different from said first locations.

10. The method of claim 9 including the step of joining said scrambled segments to procaryotic plasmids.

11. The method of claim 9 or 10 in which said step of dividing DNA into first segments is conducted in a solution of said DNA and including the step of halting the division of said DNA in successive increments of said solution over a period of time whereby to obtain a size distribution of DNA segments.

12. The method of claim 11 in which said solution contains an endonuclease that is sequence non-specific and said halting step comprises treating said successive increments to terminate the DNA cutting ability of said endonuclease.

13. The method of claim 12 in which said endonuclease is a DNase requiring a metal ion for enzyme activation, said DNA solution containing said metal salt, and in which said successive increments are obtained by continuous transfer of said solution, over said period of time, to a vessel containing a reagent comprising a chelating agent for said metal ion serving to terminate the cutting.effectiveness of said endonuclease.

56

14. Eucaryotically active DNA, comprising:

a circular molecule of DNA containing a substantially palindromic eucaryotic DNA segment serving as a eucaryotic replication origin sequence.

15. The DNA of claim 14 containing a substantially palindromic DNA segment at least 25 base pairs long and different in sequence from said eucaryotic replication origin, whereby said DNA molecule has enhanced recombinant properties.

16. A procaryotic plasmid containing a recombinant DNA segment obtained by the steps comprising:

dividing DNA into first segments at first DNA sequence locations;

joining said first segments; and

dividing said joined segments into other segments at DNA sequence locations different from said first locations.

17. DNA having enhanced recombinant properties, comprising a circular molecule of DNA containing a substantially palindromic DNA segment at least 25 base pairs long.

18. The DNA of claim 17 in which said palindromic sequence is eucaryotic.

19. A method for causing a phenotypic change in eucaryotic cells, comprising inserting into said eucaryotic cells recombinant DNA prepared in accordance with the method of any one of claims 1-13.

20. A method for causing a phenotypic change in eucaryotic cells, comprising inserting into said eucaryotic cells the recombinant DNA of any one of claims 14-18.

FIG. 1

FIG. 2

FIG. 3